# EUROPEAN PATENT SPECIFICATION

(11) **EP 2 571 380 B1**
(45) Date of publication and mention of the grant of the patent: **20.04.2016**
(21) Application number: 11723714.9
(22) Date of filing: 17.05.2011
(51) Int. Cl.: A23L 27/20, C07D 307/06

(54) **5-Alkenyl-2-oxo-tetrahydrofurane derivatives as flavoring compounds**
5-Alkenyl-2-oxo-tetrahydrofuran Derivate als Geschmacksstoffe
Derivés de 5-alcenyl-2-oxo-tetrahydrofurane comme des aromates

(30) Priority: 18.05.2010 EP 10163104
(43) Date of publication of application: 27.03.2013
(73) Proprietor: Firmenich SA, 1211 Geneva 8 (CH)
(72) Inventor: AEBERHARDT, Kasia, CH-1211 Geneva 8 (CH); FREROT, Eric, CH-1211 Geneva 8 (CH); SARRAZIN, Elise, 75017 Paris (FR)
(74) Representative: HGF Limited
(86) International application number: PCT/IB2011/052153
(87) International publication number: WO 2011/145048

(56) References cited:
- EP-A1- 0 528 044
- WO-A1-2006/048550
- JP-A- 7 118 254
- JP-A- 62 123 183
- JP-A- 2001 112 431
- US-A- 5 023 347
- FUKUSAKI, E.; SENDA, S.; NAKAZONO, Y.; OMATA, T.: "Synthesis of the Enantiomers of (Z)-5-(1-Octenyl)oxacyclopentan-2-one, a Sex Pheromone of the Cupreous Beetle, Anomala cuprea Hope", BIOSCIENCE, BIOTECHNOLOGY AND BIOCHEMISTRY, vol. 56, no. 7, 1992, pages 1160-1161, XP002589666, ISSN: 0916-8451
- SOARES LEAL, W.: "Enantiomeric anosmia in scarab beetles", JOURNAL OF CHEMICAL ECOLOGY, vol. 25, no. 5, 1999, pages 1055-1066, XP002589667, ISSN: 0098-0331
- JEROEN S DICKSCHAT ET AL: "The Chafer Pheromone Buibuilactone and Ant Pyrazines are also Produced by Marine Bacteria", JOURNAL OF CHEMICAL ECOLOGY, KLUWER ACADEMIC PUBLISHERS-PLENUM PUBLISHERS, NE LNKD- DOI:10.1007/S10886-005-3553-9, vol. 31, no. 4, 1 April 2005 (2005-04-01), pages 925-947, XP019370485, ISSN: 1573-1561

## Description

### Technical field

The present invention relates to the field of flavors. More particularly, it concerns new mono- and di-unsaturated γ-lactones and the use of mono-unsaturated γ-lactones as flavoring ingredients to impart or reinforce creamy, buttery, fatty, milky and/or green flavors and notes.

### Prior art

γ-lactones have been described in various publications such as Bedoukian, P.Z. Perfumery and Flavoring Synthetics 3rd Edition 1986, 256-265.Carol Stream, IL Editor; Novel synthesis of flavour quality γ-lactones. Zope, D.D.; Patnekar, S.G.; Kanetkar, V.R. Flavour Fragr. J. 2009, 21, 395-399; and Identification and synthesis of new γ-lactones from tuberose absolute (*Polienthes tuberosa*),. Maurer, B.; Hauser, A. Helv.Chim.Acta 1982, 65, 462-476.

γ-lactones have also been described for their properties as a pheromone. EP-A1-0528044 discloses a method for producing 4-substituted γ-lactones that are used as a pheromone trap for undesirable insects. The publication "Synthesis of the Enantiomers of (Z)-5-(1-octenyl)oxacyclopentan-2-one, a Sex Pheromone of the Cupreous Beetle, Anomala Cuprea Hope" Bioscience, Biotechnology and Biochemistry, vol 56, no. 7, 1992, pages 1160-1161, describes pheromones consisting of γ-lactones that are isolated from the cupreous chafer beetle.

The γ-lactone, 7-decene-4-olide, is described in another publication, JP-07118254 (Toray Ind Inc), where it is disclosed that the compound can be used in a perfuming composition to provide a milk-like, butter-like, fruit-like or jasmine-like fragrance, swelling and sweetness. Nevertheless, there is no suggestion in this document that a γ-lactone having both a different alkenyl chain length as well as unsaturation at a different carbon position can provide highly desirable flavors or flavor notes.

It would be desirable to provide a class of γ-lactones that are hitherto unknown for providing creamy, buttery, green, milky and/or fatty flavors and notes.

### Summary of the invention

Thus, according to the present invention there is provided the use of a compound selected from the group consisting of (E)-9-Dodecen-4-olide, (E,Z)-5,9-Dodecadien-4-olide, (Z)-9-Dodecen-4-olide, and mixtures thereof to provide or enhance the buttery or creamy flavor or note in a flavor composition or foodstuff.

Also described herein are compounds of formula (I): 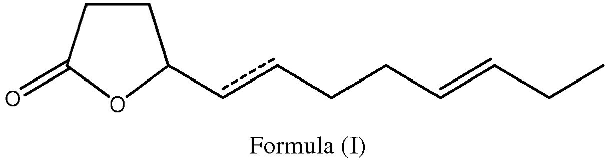 wherein the dotted line optionally represents a double bond.

Also described herein is the use of a compound of formula (II) to provide or enhance the buttery, creamy, green, fatty and/or milky flavor or note in a flavor composition or foodstuff:

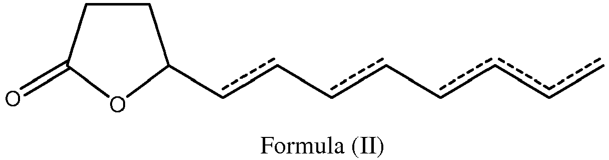

The invention additionally provides the use of a compound selected from the group consisting of (E)-9-Dodecen-4-olide, (E,Z)-5,9-Dodecadien-4-olide, (Z)-9-Dodecen-4-olide, and mixtures thereof to provide creamy, buttery, green, milky and/or fatty flavors or notes wherein the compound is used in combination with a foodstuff base.

### Detailed Description of the invention

Described herein are compounds according to formulae (I) and (II) that are suitable for use as flavoring ingredients, preferably for imparting creamy, buttery and/or fatty notes. Preferably the compounds according to the invention are used to provide a buttery or creamy flavor or note.

Notably saturated γ-lactones generally provide fruity-peachy notes, whereas δ-lactones are more reminiscent of coconut. By contrast, the γ-lactones according to the present invention change dramatically the odor toward a more buttery note, often accompanied by green undertones. These lactones are also found to deliver a particularly long-lasting flavor impact.

Compounds described herein having a one double bond in the alkyl chain may be present in the cis or trans configuration or may comprise a mixture thereof.

Compounds according to formula (II) described herein that comprise 2 double bonds in the alkyl chain, may adopt the cis, cis or cis, trans or trans, cis or trans, trans configuration or may comprise a mixture thereof.

Compounds according to formula (I) described herein include 9-Dodecen-4-olide, and 5,9-Dodecadien-4-olide, and mixtures thereof.

The compounds of the invention having the following isomeric structures are particularly preferred: (E)-9-Dodecen-4-olide, (E,Z)-5,9-Dodecadien-4-olide, (Z)-9-Dodecen-4-olide, and mixtures thereof. Of these, (E)-9-Dodecen-4-olide, (Z)-9-Dodecen-4-olide, and mixtures thereof are even more preferred.

Compounds according to formula (II) described herein include 7-Dodecen-4-olide, 9-Dodecen-4-olide, 10-Dodecen-4-olide, 10-Dodecen-4-olide, 5-Dodecen-4-olide, 5,9-Dodecadien-4-olide, 11-Dodecen-4-olide, 9-Dodecen-4-olide, and mixtures thereof.

The invention also provides a method of conferring, enhancing, improving or modifying the flavor properties, as indicated above, of a flavoring composition or of a flavored article, which method comprises adding to said composition or article an effective amount of at least a compound according to the invention. The phrase "use of a compound according to the invention" also denotes the use of any composition containing compound (I) and which can be advantageously employed in the flavor industry as active ingredients.

Also described herein is a flavoring composition comprising:
i) as flavoring ingredient, at least one compound according to the invention as defined above;
ii) at least one ingredient selected from the group consisting of a flavor carrier and a flavor base; and
iii) optionally at least one flavor adjuvant.

By "flavor carrier" we mean here a material which is practically neutral from a flavor point of view, such that it does not significantly alter the organoleptic properties of flavoring ingredients. Said carrier may be a liquid or a solid.

As a liquid carrier one may cite, as non-limiting examples, an emulsifying system, i.e. a solvent and a surfactant system, or a solvent commonly used in flavors. A detailed description of the nature and type of solvents commonly used in flavor cannot be exhaustive. However, one can cite as a non-limiting example solvents, such as propylene glycol, triacetine, triethyl citrate, benzylic alcohol, ethanol, vegetal oils or terpenes.

As solid carrier one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualitat, Behr's VerlagGmbH & Co., Hamburg, 1996. The encapsulation is a well known process to a person skilled in the art, and may be performed, for instance, using techniques such as spray-drying, agglomeration or yet extrusion; or consists of a coating encapsulation, including coacervation and complex coacervation techniques.

By "flavor base" we mean here a composition comprising at least one flavoring co-ingredient.

Said flavoring co-ingredient is not of a compound according to formulae (I) or (II). Moreover, by "flavoring co-ingredient" it is meant here a compound, which is used in flavoring preparations or compositions to impart a hedonic effect. In other words such a co-ingredient, to be considered as being a flavoring one, must be recognized by a person skilled in the art as being able to impart or modify in a positive or pleasant way the taste of a composition, and not just as having a taste.

The nature and type of the flavoring co-ingredients present in the base do not warrant a more detailed description here, which in any case would not be exhaustive, the skilled person being able to select them on the basis of its general knowledge and according to intended use or application and the desired organoleptic effect. In general terms, these flavoring co-ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of flavor. It is also understood that said co-ingredients may also be compounds known to release in a controlled manner various types of flavoring compounds.

By "flavor adjuvant" we mean here an ingredient capable of imparting additional added benefit such as a color, a particular light resistance, chemical stability, etc. A detailed description of the nature and type of adjuvant commonly used in flavoring bases cannot be exhaustive, but it has to be mentioned that said ingredients are well known to a person skilled in the art.

An invention's composition consisting of at least one compound according to the invention and at least one flavor carrier represents a particular embodiment of the invention as well as a flavoring composition comprising at least one compound according to the invention, at least one flavor carrier, at least one flavor base, and optionally at least one flavor adjuvant.

It is useful to mention here that the possibility to have, in the compositions mentioned above, more than one compound according to the invention is important as it enables the flavorist to prepare accords, flavors, possessing the flavor tonality of various compounds of the invention, creating thus new tools for his work.

Preferably, any mixture resulting directly from a chemical synthesis, e.g. without an adequate purification, in which the compound of the invention would be involved as a starting or intermediate product could not be considered as a flavoring composition according to the invention.

Moreover, a compound according to the invention can be advantageously incorporated into flavored articles to positively impart, or modify, the taste of said articles. Consequently, a flavored article comprising:
i) as flavoring ingredient, at least one compound according to the invention, as defined above, or an invention's flavoring composition; and
ii) a foodstuff base;
is also an object of the present invention.

Suitable foodstuffs, e.g. foods or beverages, include savory cubes, instant soup, canned soups, preserved meat, instant noodles, frozen dishes and preparations, sauces in all forms, flavored oils and spreads, snacks and biscuits.

For the purpose of the present invention, "foodstuff base" means an edible product, e.g. a food or a beverage. Therefore, a flavored article according to the invention comprises the functional formulation, as well as optionally additional benefit agents, corresponding to a desired edible product, e.g. a savory cube, and a flavor effective amount of at least one compound according to the invention.

The nature and type of the constituents of the foodstuffs or beverages do not warrant a more detailed description here, which in any case would not be exhaustive, the skilled person being able to select them on the basis of his general knowledge and according to the nature of said product. Nevertheless, a foodstuff such as a frozen dish or preparation, a sauce, a flavored oil, a spread, a snack or a biscuit are particularly preferred.

The proportions in which the compound according to the invention can be incorporated into the various aforementioned articles or compositions vary within a wide range of values. These values are dependent on the nature of the article to be flavored and on the desired organoleptic effect as well as the nature of the co-ingredients in a given base when the compounds according to the invention are mixed with flavoring co-ingredients, solvents or additives commonly used in the art.

In the case of flavoring compositions, typical concentrations are in the order of 0.0001 % to 1 % by weight, or even more, of the compounds of the invention based on the weight of the consumer product into which they are incorporated. Concentrations lower than these, such as in the order of 0.001% to 0.5% by weight, can be used when these compounds are incorporated into flavored articles, percentage being relative to the weight of the article.

### Examples

The invention will now be described in further detail by way of the following example, wherein the abbreviations have the usual meaning in the art.

### Example 1

### Preparation of starting materials

The following commercially available reagents and solvents were purchased from Sigma-Aldrich, Germany - titanium(IV) chloride, 1.0 M in dichloromethane (Sigma Aldrich 249866), titanium(IV) tert-butoxide (Aldrich 462551), [(1-ethoxycyclopropyl)-oxy]trimethylsilane (Aldrich 332739). 3-Ethoxy-3-oxopropylzinc bromide (Aldrich 498521); Acros Organics, USA; Carlo-Erba, France and Fluka, Switzerland. Dess-Martin periodinane as a 15% solution in dichloromethane was purchased from Acros Organics, USA (Acros 333110500). The following compounds were obtained from Firmenich SA, Switzerland - (Z,Z)-3,6-nonadienol (ref. 967327, 95% purity), 8-nonenal (obtained from Novenal DIPG, ref 967415, by dilution in cyclohexane, washing with water and then distillation, 94% purity), (Z)-6-nonenal (ref 925050, 95% purity) and (E)-3-nonenal (ref. 57652, 99% purity).

NMR spectra were processed using Bruker NMR software TopSpin 2.0 (s, singlet; d, doublet; t, triplet; m, multiplet).

For Gas Chromatography/Electron Impact-Mass Spectrometry (GC/EI-MS), the apolar column analyses were carried out using an Agilent 6890/5973 GC-MS equipped with a sniffing-port under the following conditions: column: HP1 column, 60 m, 0.32 mm I.D., 1 µm film thickness, carrier gas: helium, 5.0 mL/min, oven: 50°C, 5 min. isotherm, then gradient 3°C/min to 120°C, then gradient 5°C/min to 250°C during 5min, and to finish, gradient 15 °C/min to 300°C, followed by a 20 min isotherm, injection parameters: split ratio 1/1, injection volume 2.0 µL, detection parameters: mass spectra generated at 70 eV, scan mode ( m/z: 30-550). GC-MS-sniffing analyses were carried out by three judges including one flavorist. For the polar column, analyses were using an Agilent 6890/5973 under the following conditions: column: SupelcoWax (30 m, 0.25 mm I.D., 0.25 µm film thickness), carrier gas: helium, 0.7 mL/min, oven: 50°C, 5 min. isotherm, then gradient 3°C/min to 240°C during 15min, injection parameters: split ratio 1/1, injection volume 1.0 µL, detection parameters: mass spectra generated at 70 eV, scan mode (m/z: 30-550). GC-MS peaks were identified and integrated using HP ChemStation software.

Gas Chromatography/Flame ionization detector (GC/FID) was performed on a *Variant* GC3800. The column was a *SPB-1* 30 m, 0.25 mm I.D., 0.25 µm film thickness (*Supelco*). Oven program: 70°C, 0.5 min then 70-200°C at 10°C/min under a constant pressure of 12 psi of Helium gas. Injector was at 250°C.

### (Z,Z)-3,6-Noandieanal:

To a solution of the 5.85 g of (Z,Z)-3,6-nonadienol (41.9 mmol) in 60 mL of dichloromethane was added 207 g of the Dess-Martin periodinane solution (1.76 equiv.). The reaction was stirred for 30 min at 20°C, and then poured into a 5% NaOH solution (500 mL). The mixture was extracted 3 times in diethyl ether which was washed with brine, water, dried and evaporated. The crude product was submitted to bulb-to-bulb distillation to yield 3.7 g of a pale yellow oil (63.5% yield, 79% of (Z,Z)-3,6-nonadienal, also contains (E,Z)-2,6-nonadienal).

### (E)-7-Nonenal and (Z)-7-nonenal:

10 g of 8-Nonenal was reacted at 70°C for 6 h with 5 mg of rhodium trichloride monohydrate and 100 µL of methanol. The mixture was diluted in 200 mL of diethyl ether and washed with water, dried and evaporated. The crude product was distilled over a Vigreux column under vacuum (b.p. 40°C/0.7 mmHg) to yield 7.2 g (72%) of a mixture of (Z)- and (E)-7-nonenal in a ratio of about 1/1 together with 8% of remaining 8-nonenal and some heavier unidentified products. 4.5 g of the mixture were purified over 300 g of silica gel containing 4% of silver nitrate (eluted with 2.5% ethyl acetate in cyclohexane) to yield 2.2 g of (E)-7-nonenal (purity 95% in GC-FID, 98% (E), 2% (Z)) and 1.7 g of (Z)-7-nonenal (purity 80% in GC-FID, 96% (Z), 4% (E), contained 17% of remaining 8-nonenal).

### Spectral data for (E)-7-nonenal:

¹H NMR (δ in ppm, CDCl₃) 9.76 (t, J = 1.9 Hz, 1H), 5.42-5.39 (m, 2H), 2.44-2.40 (m, 2H), 2.00-1.95 (m, 2H), 1.66-1.60 (m, 5H), 1.40-1.28 (m, 4H).

¹³C NMR: 202.9 (d, C(1)), 131.2 (d, C(7)), 125.0 (d, C(8)), 43.9 (t, C(2)), 32.3 (t, C(6)), 29.3, 28.7 (2t, C(4,5)), 22.0 (t, C(3)), 17.9 (q, C(9)).

EI-MS, m/z(rel.intensity): 140(0.4), 122(17), 107(10), 98(23), 93(23), 83(22), 81(35), 79(27), 67(37), 55(100), 41(57), 39(31).

### Spectral data for (Z)-7-nonenal:

¹H NMR (δ in ppm, CDCl₃) 9.76 (t, J = 1.9 Hz, 1H), 5.48-5.40 (m, 1H), 5.40-5.33 (m, 1H), 2.44-2.41 (m, 2H), 2.06-2.02 (m, 2H), 1.67-1.61 (m, 2H), 1.61-1.58 (m, 3H), 1.41-1.31 (m, 4H).

¹³C NMR 202.8 (d, C(1)), 130.4 (d, C(7)), 124.0 (d, C(8)), 43.9 (t, C(2)), 29.2, 28.8 (2t, C(4,5)), 26.6 (t, C(6)), 22.0 (t, C(3)), 12.8 (q, C(9)).

EI-MS, m/z(rel.intensity): 140(0.4), 122(20), 107(12), 98(24), 93(29), 83(25), 81(41), 79(32), 67(44), 55(100), 41(71), 39(37).

### (E)-6-Nonenal:

8 g of (Z)-6-Nonenal and 0.25 g of aluminum nitrate were reacted for 6 h at 130°C. The mixture was distilled over a *Vigreux* column (b.p. 30°C/0.03 mm Hg) to yield 4.73 g (59%) of a mixture of (E)- and (Z)-6-nonenal in a ratio 65/35. The purification over AgNO₃-SiO₂ was performed under the same conditions as above, yielding 1.2 g of (E)-6-nonenal (purity 98% in GC-FID, around 1% (Z)).

¹H NMR (δ in ppm, CDCl₃): 9.76 (t, J = 1.9 Hz, 1H), 5.50-5.33 (m, 2H), 2.42 (dt, J = 7.2, 1.9 Hz, 2H), 2.03-1.96 (m, 4H), 1.68-1.60 (m, 2H), 1.43-1.36 (m, 2H), 0.96 (t, J = 7.5 Hz, 3H).

¹³C NMR : 202.8 (d, C(1)), 132.6 (d, C(7)), 128.4 (d, C(6)), 43.8 (t, C(2)), 32.2 (t, C(5)), 29.1 (t, C(4)), 25.6 (t, C(8)), 21.6 (t, C(3)), 13.9 (q, C(9)).

EI-MS, m/z(rel.intensity): 140(0.2), 122(20), 107(7), 93(25), 83(23), 81(48), 79(22), 67(51), 55(69), 54(46), 41(100), 39(35).

### (E)-4-Nonenal:

1 g of (E)-4-nonenol, was diluted in 10 mL of CH₂Cl₂. 35 g of Dess-Martin periodinane solution was added over 2 h. The reaction was stirred for an additional 2 h at 20°C, and then poured onto 50 mL of cold 5% NaOH. The mixture was extracted 3 times in diethyl ether which was washed with brine, water, dried and evaporated. The crude product was submitted to bulb-to-bulb distillation to yield 0.64 g of pale yellow oil (65% yield, purity 91% in GC-FID, 99% (E), 1% (Z)).

¹H NMR (δ in ppm, CDCl₃): 9.76 (t, J = 1.8 Hz, 1H), 5.51-5.36 (m, 2H), 2.51-2.47 (m, 2H), 2.36-2.30 (m, 2H), 2.00-1.96 (m, 2H), 1.37-1.25 (m, 4H), 0.88 (t, J = 7.2 Hz, 3H).

¹³C NMR : 202.4 (d, C(1)), 132.1 (d, C(5)), 127.6 (d, C(4)), 43.6 (t, C(2)), 32.2 (t, C(6)), 31.6 (t, C(7)), 25.2 (t, C(3)), 22.2 (t, C(8)), 13.9 (q, C(9)).

EI-MS, m/z(rel.intensity): 140(0.4), 122(11), 98(22), 97(21), 96(34), 84(88), 83(58), 81(40), 79(22), 69(36), 67(49), 55(84), 54(50), 41(100), 39(38).

### Example 2

### Synthesis of γ-Lactones according to the invention

The following general procedure was used in the preparation of the lactones. The aldehyde used in each reaction is identified below.

[(1-ethoxycyclopropyl)-oxy]-trimethylsilane (2.2 equiv.) was added over 25 min to a solution of titanium tetrachloride (2 equiv.). A deep red color showed up after 5 minutes of stirring at 20°C. Titanium (IV) tert-butoxide (1 equiv.) was added and the reaction was stirred for 1 h. Then, the aldehyde (1 equiv.) was added over 3 h using a syringe pump, at a slow and steady addition rate. The reaction was stirred for an additional 1 h, then poured onto ice with some 5% NaHCO₃, extracted two times with dichloromethane, dried and evaporated. The crude product was 4-hydroxy-ethyl ester which degraded into the lactone in the GC injector. The crude hydroxy ester was diluted to about 0.1M in methanol/5% NaOH 1/1 and the reaction was stirred overnight. Methanol was evaporated and the water phase was washed with ether, acidified to pH 1 with 5% KHSO₄ and finally extracted three times with diethyl ether. The lactones so-obtained were purified by distillation under vacuum or by flash-chromatography.
All the following lactones consisted of racemic mixtures.

### (Z)-9-Dodecen-4-olide

From 5.6 g (40 mmol) of (Z)-6-nonenal. Purified by distillation over a *Vigreux* column: b.p. 77°C/0.018 mmHg. Yield: 38%, 99% in GC-FID, 94% (Z) and 6% (E).

¹H NMR (δ in ppm, CDCl₃) 5.41-5.27 (m, 2H), 4.52-4.45 (m, 1H), 2.55-2.51 (m, 2H), 2.36-2.28 (m, 1H), 2.07-1.99 (m, 4H), 1.90-1.80 (m, 1H), 1.78-1.70 (m, 1H), 1.64-1.56 (m, 1H), 1.52-1.45 (m, 1H), 1.44-1.35 (m, 3H), 0.96 (t, J = 7.6 Hz, 3H).

EI-MS, m/z(rel.intensity): 196(7), 136(53), 123(21), 121(22), 109(18), 107(16), 95(38), 85(58), 81(78), 68(92), 67(100), 55(47), 41(56).

### 11-Dodecen-4-olide (not a compound of the invention)

From 5.6 g of 8-nonenal (40 mmol). Purified by distillation over a *Vigreux* column: b.p. 93°C/0.026 mmHg. Yield: 77%, 95% in GC-FID.

¹H NMR (δ in ppm, CDCl₃) 5.85-5.75 (m, 1H-C(11)), 4.99 (dd, J = 17.1, 1.7 Hz, 1H-C(12)), 4.93 (dd, J = 10.2, 1.3 Hz, 1H-C(12)), 4.52-4.45 (m, 1H-C(4)), 2.55-2.51 (m, 2H), 2.36-2.28(m, 1H-C(3)), 2.07-2.01 (m, 2H-C(9)), 1.90-1.80 (m, 1H), 1.77-1.69(m, 1H), 1.65-1.55(m,lH), 1.50-1.43(m, 1H), 1.41-1.30 (m, 7H).

EI-MS, m/z(rel.intensity): 196(0.1), 136(22), 95(28), 85(100), 81(40), 68(52), 67(51), 55(72), 54(61), 41(88), 39(47).

### (E,Z)-5,9-Dodecadien-4-olide

From 4.98 g (36 mmol) of (E,Z)-2,6-nonadienal. Purified by distillation over a *Vigreux* column: b.p. 94°C/0.027 mmHg. Yield: 36%, 98% in GC-FID.

¹H NMR (δ in ppm, CDCl₃): 5.85-5.78 (m, 1H-C(6)), 5.52 (dd, J = 15.3, 7.1 Hz, 1H-C(5)), 5.43-5.37 (m, 1H-C(10)), 5.33-5.27 (m, 1H-C(9)), 4.90 (dd, J = 7.3, 7.1 Hz, 1H-C(4)), 2.56-2.51 (m, 2H), 2.43-2.33 (m, 1H), 2.16-2.09 (m, 4H), 2.06-1.99 (m, 2H), 1.98-1.93 (m, 1H), 0.96 (t, J = 7.6 Hz, 3H-C(12)).

EI-MS, m/z(rel.intensity): 194 (0.1), 126(30), 84(17), 81(36), 79(19), 69(37), 68(31), 67(27), 55(21), 53(16), 41(100), 39(32).

### (E)-6-Dodecen-4-olide (not a compound of the invention)

From 1.0 g (7.1 mmol) of (E)-3-nonenal. Purified by bulb-to-bulb distillation. Yield: 42%, 90% in GC-FID.

¹H NMR (δ in ppm, CDCl₃) 5.61-5.53 (m, 1H-C(7)), 5.41-5.33 (m, 1H-C(6)), 4.56-4.49 (m, 1H-C(4)), 2.55-2.51 (m, 2H), 2.48-2.24 (m, 3H), 2.04-1.98 (m, 2H), 1.97-1.87 (m, 1H), 1.39-1.21 (m, 6H), 0.88 (t, J = 6.9 Hz, 3H).

EI-MS, m/z(rel.intensity): 196(1), 136(1), 96(3), 85(100), 57(7), 55(8), 41(15), 39(8).

### (E)-5-Dodecen-4-olide (not a compound of the invention)

From 5.0 g (36 mmol) of (E)-2-nonenal. Purified by distillation over a *Vigreux* column: b.p. 65°C/0.027 mmHg. Yield: 37%, 93% in GC-FID.

¹H NMR (δ in ppm, CDCl₃) 5.85-5.77 (m, 1H-C(6)), 5.52-5.46 (ddt, J = 14.8, 7.1, 1.7 Hz, 1H-C(5)), 4.89 (dd, J = 7.3, 7.1 Hz, 1H-C(4)), 2.56-2.51 (m, 2H), 2.41-2.33 (m, 1H), 2.09-2.03 (m, 2H), 2.02-1.93 (m, 1H), 1.42-1.35 (m, 2H), 1.33-1.25 (m, 6H), 0.88 (t, J = 6.9 Hz, 3H).

EI-MS, m/z(rel.intensity): 196(1), 153(6), 136(10), 125(14), 111(100), 98(19), 85(19), 81(24), 67(19), 55(28), 41(28).

### (E)-10-Dodecen-4-olide (not a compound of the invention)

From 1.55 g (11 mmol) of (E)-7-nonenal. Purified by distillation over a *Vigreux* column: b.p. 78°C/0.019 mmHg. Yield: 41%, 91% in GC-FID, <2% (Z).

¹H NMR (δ in ppm, CDCl₃) 5.45-5.38 (m, 2H), 4.52-4.45 (m, 1H), 2.56-2.51 (m, 2H), 2.36-2.28 (m, 1H), 2.00-1.94 (m, 2H), 1.90-1.80 (m, 1H), 1.77-1.68 (m, 1H), 1.65-1.63 (m, 3H), 1.62-1.55 (m, 1H), 1.51-1.41 (m, 1H), 1.38-1.31 (m, 5H).

EI-MS, m/z(rel.intensity): 196(5), 178(2), 167(2), 154(6), 136(42), 123(15), 109(17), 95(37), 85(66), 81(67), 68(100), 67(72), 55(79), 54(44), 41(44), 39(23).

### (Z)-10-Dodecen-4-olide (not a compound of the invention)

From 1.55 g (11 mmol) of (Z)-7-nonenal. Purified by distillation over a *Vigreux* column: b.p. 74°C/0.014 mmHg. Yield: 25%, 91% in GC-FID, <4% (E).

¹H NMR (δ in ppm, CDCIs): 5.48-5.33(m, 2H), 4.52-4.45 (m, 1H), 2.55-2.51 (m, 2H), 2.36-2.28 (m, 1H), 2.07-2.01 (m, 2H), 1.90-1.80 (m, 1H), 1.78-1.70 (m, 1H), 1.65-1.62 (m, 1H), 1.62-1.59 (m, 3H), 1.52-1.42 (m, 1H), 1.41-1.32 (m, 7H).

EI-MS, m/z(rel.intensity): 196(3), 178(2), 167(2), 154(5), 153(5), 136(36), 123(13), 109(16), 95(34), 85(62), 81(64), 68(100); 67(72), 55(73), 54(46), 41(48), 39(24).

### (E)-9-Dodecen-4-olide

From 1.13 g (7.3 mmol) of (E)-6-nonenal. Purified by micro-distillation (no *Vigreux* column): b.p. 72°C/0.024 mmHg. Yield: 26%, 91% in GC-FID, <1% (Z).

¹H NMR (δ in ppm, CDCl₃) : 5.49-5.33 (m, 2H), 4.52-4.45 (m, 1H), 2.55-2.51 (m, 2H), 2.37-2.28 (m, 1H), 2.03-1.96 (m, 4H), 1.90-1.80 (m, 1H), 1.78-1.70 (m, 1H), 1.68-1.55 (m, 1H), 1.50-1.43 (m, 1H), 1.43-1.36 (m, 3H), 0.96 (t, J = 7.4 Hz, 3H).

EI-MS, m/z(rel.intensity): 196(5), 178(2), 167(2), 154(5), 109(14), 107(14), 95(33), 85(51), 81(70), 68(96). 67(100), 55(53), 54(32), 41(75), 39(24).

### (E)-7-Dodecen-4-olide (not a compound of the invention)

From 0.6 g (11 mmol) of (E)-4-nonenal. Purified by distillation over a *Vigreux* column: b.p. 78°C/0.019 mmHg. Yield: 41%, 91% in GC-FID, around 1% (Z).

¹H NMR (δ in ppm, CDCl₃): 5.50-5.34 (m, 2H), 4.53-4.46 (m, 1H), 2.55-2.51 (m, 2H), 2.36-2.28 (m, 1H), 2.21-2.06 (m, 2H), 2.01-1.96 (m, 2H), 1.91-1.77 (m, 2H), 1.69-1.60 (m, 1H), 1.37-1.25 (m, 4H), 0.89 (t, J = 7.1 Hz, 3H).

EI-MS, m/z(rel.intensity): 196(2), 136(5), 121(4), 111(9), 96(100), 85(43), 81(61), 67(40), 55(37), 54(48), 41(31), 39(13).

### Example 3

### Preparation and evaluation of compounds in a butter flavor

For the purposes of the following examples, the concentrations of the ingredients given in the tables denote parts by weight of ingredient (ppm), relative to the weight of the evaluated end-product, whether it is a solution, or a ready-to-eat food product.

The following lactones were tested in application.

**Table 1**

| Compound |
|---|
| (Z)-9-Dodecen-4-olide ("compound 1") |
| 11-Dodecen-4-olide ("compound 2") |
| (E,Z)-5,9-Dodecadien-4-olide ("compound 3") |
| (E)-5-Dodecen-4-olide ("compound 4") |
| (E)-10-Dodecen-4-olide ("compound 5") |
| (Z)-10-Dodecen-4-olide ("compound 6") |
| (E)-9-Dodecen-4-olide ("compound 7") |
| (E)-7-Dodecen-4-olide ("compound 8") |

To an aqueous solution containing 10 ppm of a commercially available lactone key, each of the above lactone compounds was added, in the proportions indicated. The resulting flavored samples are denoted B to E (sample A being the control) below: 5

**Table 2**

| Ingredient | A | B | C | D |
|---|---|---|---|---|
| Lactone key | 10 | 10 | 10 | 10 |
| Compound 1 | | 5 | | |
| Compound 4 | | | 5 | |
| Compound 7 | | | | 5 |

| | | | | |
|---|---|---|---|---|
| (1) lactone key, 706409 02709N; origin: Firmenich SA, Geneva, Switzerland | | | | |

The solutions thus obtained were then submitted for evaluation on a blind test to a panel of at least 8 expert flavorists who were asked to describe the taste of each sample. The results are given in the following table:

**Table 3**

| Sample | Taste description |
|---|---|
| B | More dairy, creamy, milky, round |
| C | Green, vegetal, seedy, creamy |
| D | More dairy, creamy, milky, round, lingering |

### Example 4

### Preparation and evaluation of compounds in spreadable margarines

Unflavored margarine, containing 60wt% of a fat phase and 40wt% of an aqueous phase, was produced according to standard practice. The fat phase comprised 75wt% of rapeseed oil and 25wt% of an interesterified fat blend (ex Danisco), to which was added beta-carotene and 0.3wt% Dimodan® R-T PEL/B-K emulsifier (exDanisco). To the water phase was added 0.5wt% salt. The 2 phases were emulsified at 50°C. The emulsion was then chilled in a series of cooling sections and crystallized according to standard processes.

Margarine was flavored by incorporating, with stirring, a selected amount of a lactone compound to prepare samples A to I, below. The product was then kept at 4°C for at least 24 hours before evaluation. The amounts are given in the table below:

**Table 4**

| Sample | A | B | C | D | E | F | G | H | I |
|---|---|---|---|---|---|---|---|---|---|
| Compound 1 | | 40 | | | | | | | |
| Compound 2 | | | 40 | | | | | | |
| Compound 3 | | | | 40 | | | | | |
| Compound 4 | | | | | 40 | | | | |
| Compound 5 | | | | | | 60 | | | |
| Compound 6 | | | | | | | 40 | | |
| Compound 7 | | | | | | | | 40 | |
| Compound 8 | | | | | | | | | 60 |

The flavored margarine samples were then evaluated by a panel of at least 8 expert flavorists in 2 blind test sessions comparing samples B to I against control sample A. The results are given in the following table:

**Table 5**

| Sample | Taste |
|---|---|
| B | Creamy, buttery, lactonic, peachy, fatty |
| C | Waxy, candle, dusty |
| D | Green, leafy, fruity, fatty, creamy, sweet |
| E | Peachy, fruity, green, cardboard |
| F | More creamy, buttery |
| G | Candle, waxy, green, synthetic |
| H | Green, animalic |
| I | Fruity, peachy, waxy, cardboard |

Product B was especially appreciated by the flavorists because of its creamy, buttery, fatty character.

### Example 5

### Preparation and evaluation of compounds in flavored margarines

The margarine base prepared in example 4 was flavored by incorporation with stirring of a butter flavor (706409 02714T; Origin: Firmenich SA, Geneva, Switzerland). The flavored margarine was then kept at 4°C for minimum 24 hours. The lactone compounds were then stirred into the flavored margarine to prepare samples A to D, below. The samples were then stored at 4°C for minimum 24 hours. The amounts are given in the following table:

**Table 6**

| Ingredient | A | B | C | D |
|---|---|---|---|---|
| Butter flavor | 30 | 30 | 30 | 30 |
| Compound 1 | | 20 | | |
| Compound 2 | | | 80 | |
| Compound 5 | | | | 20 |

The samples were then evaluated on a blind test by a panel of at least 8 expert flavorists and compared to a base sample which only contained the butter flavor (sample A).

The results are given in the following table:

**Table 7**

| Sample | Taste |
|---|---|
| B | More buttery, more creamy, more mouthfeel, more milky, dairy |
| C | Candle, waxy, green, long lasting |
| D | More mouthfeel, long lasting, more oily, more fatty |

Products B and D were especially appreciated by the flavorists because of their creamy, buttery, fatty character.

## Claims

1. Use of a compound selected from the group consisting of (E)-9-Dodecen-4-olide, (E,Z)-5,9-Dodecadien-4-olide, (Z)-9-Dodecen-4-olide, and mixtures thereof to provide or enhance the buttery or creamy flavor or note in a flavor composition or foodstuff.

2. Use of a compound as defined in claim 1, wherein the compound is used in combination with a foodstuff base.

3. Use according to claim 2, wherein the foodstuff base is a frozen dish or preparations, a sauce, a flavored oil, a spread, a snack or a biscuit.

## Patentansprüche

1. Verwendung einer Verbindung ausgewählt aus der Gruppe bestehend aus (E)-9-Dodecen-4-olid, (E,Z)-5,9-Dodecen-4-olid, (Z)-9-Dodecen-4-olid und Mischungen davon, um den buttugen oder cremigen Geschmack oder die buttuge oder cremige Note in einer Geschmackszusammensetzung oder in einem Lebensmittel bereitzustellen oder zu verbessern.

2. Verwendung einer Verbindung gemäß Anspruch 1, wobei die Verbindung in Kombination mit einer Lebensmittelbasis verwendet wird.

3. Verwendung gemäß Anspruch 2, wobei die Lebensmittelbasis ein tiefgefrorenes Gericht oder Präparate, eine Sauce, ein Geschmacksöl, ein Aufstrich, ein Snack oder ein Keks ist.

## Revendications

1. Utilisation d'un composé choisi dans le groupe composé de (E)-9-dodécène-4-olide (E,Z)-5,9-dodécadiène-4-olide, (Z)-9-dodécène-4-olide, et leurs mélanges pour donner ou améliorer une saveur ou une note crémeuse ou onctueuse dans une composition de la saveur ou une denrée alimentaire.

2. Utilisation d'un composé tel que défini dans la revendication 1, où le composé est utilisé en combinaison avec une denrée alimentaire de base.

3. Utilisation selon la revendication 2, où la denrée alimentaire de base est un plat congelé ou des préparations congelées, une sauce, une huile parfumée, une pâte à tartiner, un casse-croûte ou un biscuit.
